# EUROPÄISCHE PATENTSCHRIFT

(11) **EP 2 997 876 B1**
(45) Veröffentlichungstag und Bekanntmachung des Hinweises auf die Patenterteilung: **28.03.2018**
(21) Anmeldenummer: 15185061.7
(22) Anmeldetag: 14.09.2015
(51) Int. Cl.: A61B 1/00, A61B 1/015, F16K 11/20, G02B 23/24

(54) **INSUFFLATIONS- UND SPÜLVENTIL SOWIE ENDOSKOP MIT EINEM INSUFFLATIONS- UND SPÜLVENTIL**
INSUFFLATION AND IRRIGATION VALVE AND ENDOSCOPE COMPRISING AN INSUFFLATION AND IRRIGATION VALVE
VALVE D'INSUFFLATION ET DE VIDANGE ET ENDOSCOPE COMPRENANT UNE VALVE D'INSUFFLATION ET DE VIDANGE

(30) Priorität: 18.09.2014 DE 102014113472
(43) Veröffentlichungstag der Anmeldung: 23.03.2016
(73) Patentinhaber: Karl Storz SE & Co. KG, 78532 Tuttlingen (DE)
(72) Erfinder: Tobien, Peter, 78532 Tuttlingen (DE); Rebholz, Clemens, 78532 Tuttlingen (DE)
(74) Vertreter: Fugmann, Winfried

(56) Entgegenhaltungen:
- EP-A1- 1 707 107
- JP-A- S57 103 621
- JP-A- 2009 039 463
- JP-A- 2010 268 866
- JP-A- 2012 249 654
- US-A- 5 027 791

## Beschreibung

Die vorliegende Erfindung betrifft ein Insufflations- und Spülventil nach dem Oberbegriff von Anspruch 1 sowie ein Endoskop mit einem derartigen Insufflations- und Spülventil.

Für die Durchführung eines endoskopischen Eingriffs ist häufig die Schaffung eines körperinneren Hohlraums erforderlich. Hierfür wird üblicherweise ein Gas, beispielsweise Luft oder CO₂, von einem proximalen (benutzernahen) Endbereich eines Endoskops durch eine innerhalb des Schafts des Endoskops verlaufende Leitung bis zum distalen (benutzerfernen) Ende des Endoskops geführt. Dort tritt es unter einem geeigneten Druck aus und schafft im Bereich des distalen Endes des Endoskops einen Hohlraum, innerhalb dessen der Eingriff unter endoskopischer Sicht durchgeführt werden kann. Da bei dem Eingriff das distale Fenster der endoskopischen Optik verschmutzt werden kann, wodurch die Sicht beeinträchtigt wird, kann durch den Schaft des Endoskops eine Spülflüssigkeit bis zum distalen Ende des Endoskops geführt werden, um mit dieser während des endoskopischen Eingriffs das distale Fenster zu reinigen. Weiterhin kann eine Absaugung vorgesehen sein, mit der die Spülflüssigkeit sowie ggf. abgetrennte Gewebeteile aus dem Operationsgebiet abgesaugt werden können.

Zur Steuerung der Insufflation und Spülung ist bei bekannten Endoskopen ein manuell betätigbares Insufflations- und Spülventil am Handgriff des Endoskops angeordnet. An ein Ventilgehäuse des bekannten Insufflations- und Spülventils ist ein proximaler Abschnitt einer Insufflationsleitung angeschlossen, über den ein Insufflationsgerät mit dem Endoskop verbunden werden kann, das das Insufflationsgas unter dem erforderlichen Druck bereitstellt. Weiterhin ist an das Ventilgehäuse ein proximaler Abschnitt einer Spülleitung angeschlossen, worin aus einem Vorratsbehälter eine Spülflüssigkeit ebenfalls unter einem geeigneten Druck bereitgestellt wird. Am Ventilgehäuse sind ferner ein distaler Abschnitt der Insufflationsleitung und ein distaler Abschnitt der Spülleitung angeschlossen, durch welche das Insufflationsgas bzw. die Spülflüssigkeit zum distalen Ende des Endoskops geleitet werden können. Das Insufflations- und Spülventil umfasst einen im Ventilgehäuse verschiebbar angeordneten Stößel, der durch einen Ventilknopf betätigt werden kann, wobei durch Verschieben des Stößels der proximale und der distale Abschnitt der Spülleitung miteinander verbunden oder voneinander getrennt werden können. Der Ventilknopf und der Stößel weisen jeweils eine Bohrung auf, die am einen Ende mit dem proximalen Abschnitt der Insufflationsleitung in Verbindung steht und am anderen Ende in einer Öffnung endet, durch die das Insufflationsgas in die Umgebung entweichen kann. Wird die Öffnung vom Benutzer mit einem Finger verschlossen, so kann sich in der Insufflationsleitung Druck aufbauen und eine Insufflation erfolgen. Wird das Insufflations- und Spülventil durch weiteren Druck mit dem Finger eingedrückt, so wird der distale mit dem proximalen Abschnitt der Spülleitung verbunden, so dass Spülflüssigkeit zum distalen Ende des Endoskops strömen kann.

Im unbetätigten Zustand des Ventils, also dann, wenn weder gespült noch insuffliert werden soll, entweicht das vom Insufflationsgerät bereitgestellte Insufflationsgas über die Öffnung in die Umgebung. Insbesondere in dem Fall, dass als Insufflationsgas CO₂ verwendet wird, ist dies mit erheblichen Kosten verbunden, da der Vorratsbehälter, etwa eine CO₂-Flasche, unnötig entleert wird und vorzeitig gewechselt werden muss.

Aus EP 1 707 107 A1 ist ein Insufflations- und Spülventil bekannt, das ein Zylinderelement mit einer näherungsweise zylindrischen Bohrung aufweist, in der ein zylindrischer äußerer Stößel verschiebbar angeordnet ist. Ein distaler Abschnitt einer Luftleitung ist mit dem Boden der Bohrung verbunden, und ein proximaler Abschnitt der Luftleitung sowie ein proximaler und ein distaler Abschnitt einer Wasserleitung sind an vorbestimmten Positionen seitlich an dem Zylinderelement angeschlossen. In einer Bohrung des äußeren Stößels ist ein innerer Stößel verschiebbar angeordnet, der in seinem Inneren einen durch Querbohrungen und eine Längsbohrung gebildeten Luftführungskanal aufweist. An der Unterseite des äußeren Stößels ist eine Grundplatte angeordnet, auf der sich eine Feder abstützt, die den inneren Stößel vorspannt. Der Luftführungskanal kommuniziert mit einem Loch in der Grundplatte und, wenn der innere Stößel manuell gegen die Kraft der Feder in den äußeren Stößel hineingedrückt wird, mit einem Durchbruch und einer umlaufenden Nut des äußeren Stößels. Die umlaufende Nut des äußeren Stößels kommuniziert in einer ersten Stellung des äußeren Stößels mit dem distalen Abschnitt der Luftleitung, so dass durch Betätigen des inneren Stößels eine Verbindung zwischen dem proximalen und dem distalen Abschnitt der Luftleitung hergestellt werden kann. In einer zweiten Stellung des äußeren Stößels wird über eine umlaufende Nut des äußeren Stößels eine Verbindung zwischen dem proximalen und dem distalen Abschnitt der Wasserleitung hergestellt.

Gemäß US 5,027,791 umfasst eine Luft- und Wasserzuführungsvorrichtung für ein Endoskop einen in einem Steuerungsteil vorgesehenen Zylinder mit jeweils ersten und zweiten Leitungen für Luft und Wasser. Ein Wasserzuführungsschaltventil ist verschiebbar in den Zylinder eingesetzt, um den Kommunikationszustand zwischen den ersten und zweiten Wasserleitungen zu verändern, und ein Luftzuführungsschaltventil ist verschiebbar in das Wasserzuführungsschaltventil eingesetzt, um den Kommunikationszustand zwischen den ersten und zweiten Luftleitungen zu verändern. Ein Körper des Wasserzuführungsschaltventils wird durch eine erste Schraubenfeder in eine Richtung nach außerhalb des Zylinders vorbelastet, und ein Körper des Luftzuführungsschaltventils wird durch eine zweite Schraubenfeder in eine Richtung nach außerhalb des Körpers des Wasserzuführungsschaltventils vorbelastet. Das in JP S57 103621 A offenbarte entsprechend der Präambel von Anspruch 1 weist einen inneren und einen äußeren Stößel auf, wobei der innere Stößel gegen die Kraft einer Feder relativ zu dem äußeren Stößel verschiebbar ist.

Da beim Betrieb des Insufflations- und Spülventils eine Verschmutzung des Ventils eintreten kann und insbesondere eine Kontamination mit Körperflüssigkeit nicht vollständig ausgeschlossen werden kann, ist bei einem wiederverwendbaren Ventil eine Reinigung und Sterilisation erforderlich. Dies ist bei dem beschriebenen Insufflations- und Spülventil jedoch nur eingeschränkt möglich, insbesondere ist die Demontage erschwert und die Reinigung der im inneren Stößel ausgebildeten Kanäle kaum möglich. Ferner verhindert die Grundplatte, an der sich die Feder abstützt, die Reinigung der zentralen Bohrung des äußeren Stößels mit einer durch diese hindurch geführten Spülbürste.

Es ist Aufgabe der vorliegenden Erfindung, ein Insufflations- und Spülventil anzugeben, das die oben genannten Nachteile nicht aufweist, das insbesondere einfacher aufgebaut und/oder einfacher zu reinigen ist, sowie ein Endoskop mit einem entsprechenden Insufflations- und Spülventil.

Diese Aufgabe wird durch ein Insufflations- und Spülventil gemäß Anspruch 1 sowie durch ein Endoskop gemäß Anspruch 10 gelöst.

Vorteilhafte Weiterbildungen der Erfindung ergeben sich aus den Unteransprüchen.

Ein erfindungsgemäßes Insufflations- und Spülventil dient zur Steuerung der Insufflation und des Spülens bei einem mit einem Endoskop durchgeführten Eingriff und ist insbesondere zur Anordnung im distalen Endbereich eines Endoskops an einem Handgriff des Endoskops vorgesehen. Das Insufflations- und Spülventil kann aber auch zur Verwendung an einer vom Endoskop getrennten Bedieneinheit ausgebildet sein. Mit dem Insufflations- und Spülventil können die Funktionen der Insufflation und der Spülung manuell gesteuert werden. Ein derartiges Ventil wird auch als "kombiniertes Ventil" oder "Doppelventil" bezeichnet.

Das Insufflations- und Spülventil umfasst ein Ventilgehäuse, das eine im Wesentlichen zylindrisch ausgebildete Bohrung aufweist, die an ihrem einen Ende mit einem Boden abgeschlossen ist. Das Ventilgehäuse kann ein separates Bauteil sein, das beispielsweise in ein Gehäuse des Handgriffs des Endoskops eingesetzt werden kann. Das Ventilgehäuse kann aber auch durch das Gehäuse des Handgriffs selbst oder durch ein anderes übergeordnetes Bauteil des Endoskops bzw. einer Bedieneinheit gebildet werden oder mit diesem einstückig ausgeführt sein.

In die Bohrung des Ventilgehäuses münden ein Gaseingang, ein Gasausgang, ein Spülmediumeingang und ein Spülmediumausgang. Die genannten Eingänge und Ausgänge können mit Anschlussstutzen verbunden sein, an die Leitungen, beispielsweise Schläuche, für das Insufflationsgas bzw. das Spülmedium angeschlossen werden können. Der Gaseingang, der Gasausgang, der Spülmediumeingang und/oder der Spülmediumausgang können aber auch mit Leitungswegen verbunden sein, die durch im Ventilgehäuse oder in einem dieses umfassenden Bauteil, etwa einem Gehäuse des Handgriffs des Endoskops, ausgebildete Hohlräume gebildet werden. Das Spülmedium ist in der Regel eine Spülflüssigkeit, beispielsweise physiologische Kochsalzlösung, kann aber auch ein gasförmiges Spülmedium sein.

Das Insufflations- und Spülventil umfasst weiterhin einen äußeren Stößel, der in der im Wesentlichen zylindrisch ausgebildeten Bohrung des Ventilgehäuses verschiebbar ist zwischen einer Offenstellung und einer Schließstellung hinsichtlich der Durchleitung des Spülmediums. In der Offenstellung besteht eine Fluidverbindung des Spülmediumeingangs mit dem Spülmediumausgang, und in der Schließstellung ist diese Verbindung unterbrochen. Unter "Fluidverbindung" wird in diesem Zusammenhang eine Verbindung verstanden, die das Strömen eines Fluids, d.h. eines Gases oder einer Flüssigkeit, ermöglicht, im Falle der Fluidverbindung des Spülmediumeingangs und des Spülmediumausgangs insbesondere das Strömen einer unter einem Druck bereitgestellten Spülflüssigkeit von einem an den Spülmediumeingang angeschlossenen proximalen Abschnitt einer Spülleitung zu einem an den Spülmediumausgang angeschlossenen distalen Abschnitt der Spülleitung. Der äußere Stößel ist manuell verschiebbar. Insbesondere kann der äußere Stößel durch eine Federkraft in die Schließstellung vorgespannt sein und gegen die Federkraft durch Fingerdruck auf einen Ventilknopf des äußeren Stößels in die Offenstellung verschiebbar sein.

Ferner umfasst das Insufflations- und Spülventil einen inneren Stößel, der in einer Längsbohrung des äußeren Stößels relativ zu diesem verschiebbar angeordnet ist. Die Längsbohrung des äußeren Stößels ist beidseitig offen und vorzugsweise zentral im äußeren Stößel angeordnet. Vorzugsweise sind der innere und der äußere Stößel hinsichtlich ihrer jeweiligen Längsachsen somit koaxial angeordnet. Der innere Stößel ist relativ zum äußeren Stößel zwischen einer auf den Boden der zylindrischen Bohrung des Ventilgehäuses bezogen bodennahen Stellung und einer bodenfernen Stellung verschiebbar. In der bodenfernen Stellung des inneren Stößels besteht keine Fluidverbindung des Gaseingangs mit dem Gasausgang. Befindet sich der innere Stößel in der bodennahen Stellung und befindet sich der äußere Stößel in der Schließstellung, in der die Fluidverbindung des Spülmediums unterbrochen ist, so besteht eine Fluidverbindung des Gaseingangs mit dem Gasausgang, d.h. ein Insufflationsgas, das in einem an den Gaseingang angeschlossenen proximalen Abschnitt einer Insufflationsleitung unter einem Druck bereitgestellt wird, kann in einen an den Gasausgang angeschlossenen distalen Abschnitt der Insufflationsleitung strömen. Befindet sich der innere Stößel in der bodennahen Stellung und befindet sich der äußere Stößel in der Offenstellung, so kann die Fluidverbindung des Gaseingangs mit dem Gasausgang unterbrochen sein. Durch Verschieben des inneren Stößels relativ zum äußeren Stößel kann somit die Fluidverbindung des Gasausgangs mit dem Gaseingang derart gesteuert werden, dass in der bodenfernen Stellung keine Fluidverbindung besteht und in der bodennahen Stellung in zumindest einer Stellung des äußeren Stößels eine Fluidverbindung besteht und somit eine Insufflation stattfinden kann.

Erfindungsgemäß mündet der Gaseingang in Bezug auf den Boden der zylindrischen Bohrung des Ventilgehäuses, in dem der äußere Stößel verschiebbar ist, bodennäher als der Gasausgang in die Bohrung. Insbesondere mündet der Gaseingang in einen Bodenbereich der Bohrung, beispielsweise in den Boden oder in einen an den Boden angrenzenden Bereich der im Wesentlichen zylindrischen Seitenwand der Bohrung. Der Gasausgang mündet in die Seitenwand der Bohrung; sofern der Gaseingang auch in die Seitenwand mündet, so mündet der Gasausgang somit an einer Position, die vom Boden der Bohrung weiter entfernt ist als die Position der Mündung des Gaseingangs.

Dadurch, dass der Gaseingang bodennäher in die Bohrung des Ventilgehäuses mündet als der Gasausgang, wird erfindungsgemäß erreicht, dass der vom Insufflationsgerät, beispielsweise einer Insufflationspumpe, erzeugte Druck des Insufflationsgases sich im Bodenbereich der zylindrischen Bohrung des Ventilgehäuses derart auswirkt, dass hierdurch der innere Stößel in die bodenferne Stellung vorbelastet wird. In der bodenfernen Stellung des inneren Stößels ist der Gasausgang vom Gaseingang getrennt. Die Normalstellung des Insufflations- und Spülventils, in der keine Insufflation stattfindet, kann erfindungsgemäß somit durch den vom Insufflationsgerät erzeugten Druck erreicht oder zumindest gehalten werden, ohne dass eine Vorbelastung des inneren Stößels durch eine Feder erforderlich ist. Der innere Stößel ist allein durch den vom Insufflationsgerät bereitgestellten Gasdruck in die bodenferne Stellung, die hinsichtlich der Insufflation die Schließstellung ist, bewegbar und kann in dieser gehalten werden. Eine Feder zur Vorbelastung des inneren Stößels in seine bodenferne Stellung ist somit nicht notwendig. Hierdurch ist eine

Vereinfachung des konstruktiven Aufbaus und der Herstellung sowie eine Erleichterung der Reinigung des Insufflations- und Spülventils erreichbar. Ferner kann ein für die Feder des inneren Stößels notwendiger Bauraum entfallen, wodurch die Baulänge des Insufflations- und Spülventils reduziert werden kann.

Gemäß einer bevorzugten Ausführungsform der Erfindung wird die Verbindung des Gaseingangs mit dem Gasausgang zur Durchführung der Insufflation über einen Verbindungskanal des äußeren Stößels, eine auf der Außenseite des inneren Stößels ausgebildete Ausnehmung und die bodenseitige Öffnung der Längsbohrung ausgebildet. Um eine weitgehend leckagefreie Fluidverbindung zu ermöglichen, kann der Verbindungskanal des äußeren Stößels in axialer Richtung beidseitig von jeweils einer Dichtung begrenzt sein, die den äußeren Stößel gegen die Wand der zylindrischen Bohrung des Ventilgehäuses abdichtet. Dadurch, dass die Verbindung zwischen dem Gaseingang und dem Gasausgang über den Verbindungskanal des äußeren Stößels, die auf der Außenseite des inneren Stößels ausgebildete Ausnehmung und die Öffnung der Längsbohrung des äußeren Stößels zum bodenseitigen Raum der zylindrischen Bohrung des Ventilgehäuses hergestellt werden kann, kann eine Fluidverbindung mit einem großen Querschnitt geschaffen werden, die einen ungehinderten Gasstrom und eine leichte Reinigung ermöglicht.

Vorzugsweise ist die auf der Außenseite des inneren Stößels ausgebildete Ausnehmung als umlaufende Nut ausgebildet oder als eine Verjüngung, die von einem bodenseitigen Endabschnitt des inneren Stößels bis zu einem dem Verbindungskanal des äußeren Stößels gegenüberliegenden Abschnitt des inneren Stößels reicht. Dadurch, dass die Ausnehmung als umlaufende Nut oder als Verjüngung ausgebildet ist, kann ein Gasstrom unabhängig von einer Drehstellung des inneren Stößels bezüglich seiner Längsachse gewährleistet werden. Hierdurch wird es ermöglicht, den inneren Stößel ohne eine Verdrehsicherung auszubilden, wodurch der Aufbau des Insufflations- und Spülventils und die Herstellung des inneren Stößels weiter vereinfacht werden; insbesondere wird es ermöglicht, den inneren Stößel als einfaches Drehteil herzustellen. Ferner wird die Reinigung des inneren Stößels vereinfacht, da alle zu reinigenden Oberflächen direkt von außen zugänglich sind.

Erfindungsgemäß ist das Insufflations- und Spülventil derart ausgebildet, dass die bodenseitige Öffnung der Längsbohrung des äußeren Stößels in der bodenfernen Stellung des inneren Stößels verschlossen ist. Hierdurch wird auf einfache Weise ermöglicht, den Gasstrom vom Gaseingang zum Gasausgang in der bodenfernen Stellung des inneren Stößels zu unterbrechen. Da der innere Stößel durch den vom Insufflationsgerät bereitgestellten Gasdruck in die bodenferne Stellung gedrückt wird, kann hierdurch auf besonders einfache Weise erreicht werden, dass der innere Stößel bei Nichtbetätigung durch einen Benutzer die bodenferne Stellung einnimmt, in der die Insufflation unterbrochen ist. Erfindungsgemäß ist das bodenseitige Ende des inneren Stößels mit einem Ventilteller zum Verschließen der bodenseitigen Öffnung der Längsbohrung ausgebildet. Insbesondere kann der innere Stößel mit der bodenseitigen Öffnung der Längsbohrung des äußeren Stößels nach Art eines Tellerventils zusammenwirken, wobei die bodenseitige Öffnung der Längsbohrung als Ventilsitz ausgebildet ist. So kann beispielsweise das bodenseitige Ende des inneren Stößels unterseitig flach und oberseitig kegelförmig ausgestaltet sein, wobei die kegelförmige Oberseite mit einer korrespondierenden kegelförmigen Einsenkung des unteren Endes des äußeren Stößels zum Abdichten der Längsbohrung des äußeren Stößels zusammenwirkt. Hierdurch kann auf einfache Weise eine besonders sichere gasdichte Unterbrechung der Fluidverbindung des Gaseingangs mit dem Gasausgang erreichbar sein, die durch den vom Insufflationsgerät bereitgestellten Gasdruck bewirkt wird.

Zur Durchführung der Insufflation ist der innere Stößel gegen den Gasdruck manuell in die Längsbohrung des äußeren Stößels hineindrückbar, so dass dieser relativ zum äußeren Stößel aus seiner bodenfernen Stellung heraus gedrückt wird und insbesondere seine bodennahe Stellung einnimmt. Hierfür weist der innere Stößel vorzugsweise eine solche Länge auf, dass in der bodenfernen Stellung des inneren Stößels ein bodenfernes Ende über eine Betätigungsfläche eines Ventilknopfs, auf die ein Benutzer zum Verschieben des äußeren Stößels einen Fingerdruck ausübt, hinausragt und in der bodennahen Stellung des inneren Stößels mit der Betätigungsfläche des Ventilknopfs im Wesentlichen bündig abschließt. Es ist somit für einen Benutzer durch Ertasten des herausstehenden Endes des inneren Stößels bzw. einer ebenen Fläche leicht wahrnehmbar, ob der innere Stößel sich außerhalb seiner bodennahen Stellung befindet oder ob die bodennahe Stellung des inneren Stößels erreicht ist. Hierdurch wird die Bedienung des Insufflations- und Spülventils vereinfacht.

Gemäß einer bevorzugten Ausführungsform der Erfindung ist in der Offenstellung des äußeren Stößels der Gasausgang verschlossen. Hierfür kann die äußere Oberfläche des äußeren Stößels zum Verschließen des Gasausgangs ausgebildet sein und beispielsweise einen entsprechenden Sperrkörper, ggf. mit diesen umschließenden Dichtungen, aufweisen. Insbesondere ist die Offenstellung des äußeren Stößels bezogen auf den Boden der zylindrischen Bohrung des Ventilgehäuses eine bodennahe Stellung des äußeren Stößels. Während gemäß dieser Ausführungsform der Erfindung in der Offenstellung des äußeren Stößels der Gasausgang somit verschlossen ist, ist in der Schließstellung des äußeren Stößels der Gasausgang durch Betätigen des inneren Stößels wahlweise mit dem Gaseingang verbindbar oder von diesem trennbar. Hierdurch wird es ermöglicht, dass in der Normalstellung, in der ein Bediener weder den äußeren Stößel noch den inneren Stößel betätigt, sowohl die Spülung als auch die Insufflation unterbrochen sind. In einer zweiten Stellung des Ventils kann der innere Stößel derart in die Längsbohrung des äußeren Stößels hineindrückbar sein, dass die Verbindung zwischen dem Gaseingang und dem Gasausgang geöffnet wird und somit das vom Insufflationsgerät bereitgestellte Gas zum distalen Ende des Endoskops geleitet werden kann. In einer dritten Stellung wird der äußere Stößel gemeinsam mit dem inneren Stößel in die zylindrische Bohrung des Ventilgehäuses hineingedrückt, wodurch gemäß dieser Ausführungsform die Spülung aktiviert und die Insufflation unterbrochen wird. In Zwischenstellungen, insbesondere in Zwischenstellungen des äußeren Stößels zwischen der Offenstellung und der Schließstellung bei in seine bodennahe Stellung hineingedrücktem inneren Stößel, kann es möglich sein, sowohl Insufflation als auch Spülung gleichzeitig durchzuführen. Hierdurch wird eine besonders einfache Bedienung beider Funktionen des Insufflations- und Spülventils ermöglicht.

Vorzugsweise wird die Fluidverbindung zwischen dem Spülmediumeingang und dem Spülmediumausgang über eine auf der Außenseite des äußeren Stößels ausgebildete umlaufende Nut hergestellt. Dabei ist die axiale Länge der umlaufenden Nut derart gewählt und diese derart angeordnet, dass die Nut nur in der Offenstellung sowohl mit dem Spülmediumeingang als auch mit dem Spülmediumausgang korrespondiert und hierdurch die Verbindung herstellt. Hierdurch wird eine Verbindung zum Durchführen der Spülung mit einem besonders großen Querschnitt ermöglicht, die zudem von einer Drehstellung des äußeren Stößels relativ zum Ventilgehäuse unabhängig sein kann.

Ferner ist es bevorzugt, dass im Bereich der Mündung des Spülmediumeingangs, des Spülmediumausgangs und/oder des Gasausgangs die im Wesentlichen zylindrische Bohrung des Ventilgehäuses durch eine jeweilige umlaufende flache Nut mit mindestens einer abgeschrägten Wand erweitert ist. Im Übrigen kann die Bohrung des Ventilgehäuses zylindrisch ausgeführt sein. Hierdurch wird ein verschleißarmes Gleiten der zur Abdichtung des äußeren Stößels gegen die zylindrische Bohrung vorgesehenen Dichtungen in der Bohrung ermöglicht.

Weiterhin ist es bevorzugt, dass der Gasausgang sowie ggf. auch der Spülmediumausgang durch jeweils einen schräg zu einer Längsachse der Bohrung des Ventilgehäuses in dieses einmündenden und insbesondere schräg zur Längsachse der Bohrung gerichteten gebildet werden. Diese können als Stutzen zum Anschließen von Leitungen oder Schläuchen ausgebildet sein. Hierdurch wird eine besonders platzsparende Bauform eines Insufflations- und Spülventils ermöglicht.

Ein erfindungsgemäßes Endoskop weist ein wie oben beschrieben ausgebildetes Insufflations- und Spülventil auf, das insbesondere am Handgriff des Endoskops angeordnet ist und von einem Benutzer des Endoskops mit einem Finger betätigt werden kann. Das Endoskop kann als starres, halbstarres oder flexibles Endoskop ausgebildet sein. Insbesondere ist das Endoskop ein Gastroskop mit einem langerstreckten flexiblen Schaft und einem am proximalen Ende des Schafts angeordneten Handgriff, der das Insufflations- und Spülventil aufweist. Der Handgriff kann weitere Bedienelemente aufweisen, beispielsweise ein Saugventil sowie Bedienelemente zum Steuern weiterer Funktionen des Endoskops. Der Gaseingang und der Spülmediumeingang können Anschlussstutzen zum Anschließen des proximalen Abschnitts einer Insufflations- und einer Spülleitung aufweisen, durch die eine Verbindung mit einem separaten Insufflationsgerät und einem Vorratsbehälter für Spülmedium, der ebenfalls mit dem vom Insufflationsgerät bereitgestellten Druck beaufschlagt wird, geschaffen werden kann. Der Gasausgang und der Spülmediumausgang des Insufflations- und Spülventils sind mit den distalen Abschnitten der Insufflations- und der Spülleitung verbunden, die durch den Schaft des Endoskops bis zu dessen distalem Ende verlaufen. Das distale Ende der Spülleitung kann auf das distale Fenster der Optik des Endoskops gerichtet sein. Innerhalb des Schafts kann eine gemeinsame Leitung für Insufflation und Spülung vorgesehen sein. Durch die erfindungsgemäße Ausbildung des Endoskops mit dem beschriebenen Insufflations- und Spülventil wird ein Endoskop geschaffen, bei dem eine einfache manuelle Steuerung von Insufflation und Spülung möglich ist und bei dem das Insufflations- und Spülventil einfach aufgebaut und leicht zu reinigen ist.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1 ein Insufflations- und Spülventil gemäß einem Ausführungsbeispiel der Erfindung in einer ersten Stellung im Längsschnitt;
Fig. 2 das Insufflations- und Spülventil der Figur 1 in einer zweiten Stellung;
Fig. 3 das Insufflations- und Spülventil der Figur 1 in einer dritten Stellung.

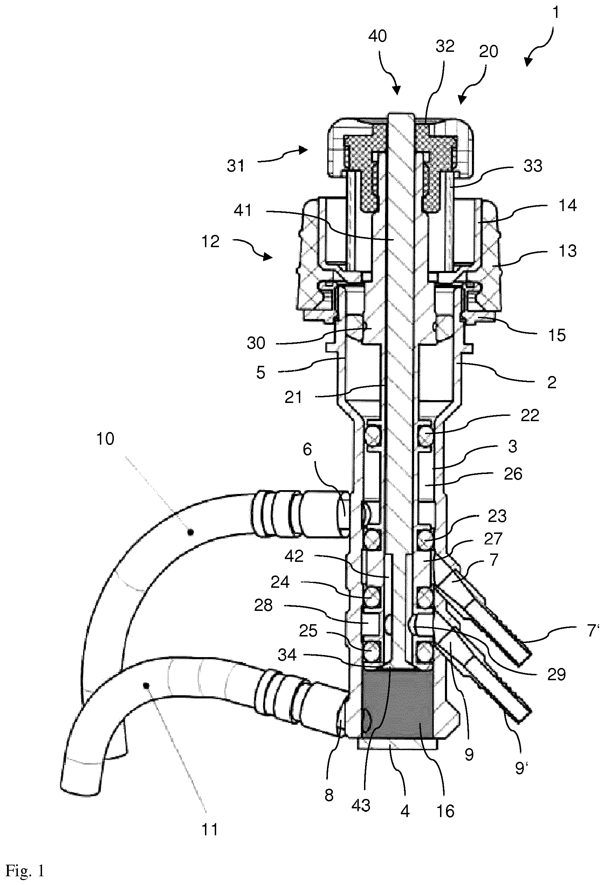

Wie in Fig. 1 gezeigt, umfasst ein Insufflations- und Spülventil 1 gemäß einem Ausführungsbeispiel der Erfindung ein Ventilgehäuse 2, das eine im Wesentlichen zylindrische Bohrung 3 aufweist. Die Bohrung 3 ist an ihrem unteren Ende durch einen Boden 4 verschlossen und geht an ihrem oberen Ende in eine Erweiterung 5 über. Der Boden 4 kann mit dem Ventilgehäuse 2 einstückig ausgebildet sein oder auch an das Ventilgehäuse 2 angesetzt sein, beispielsweise angeschraubt oder angeschweißt. Das Ventilgehäuse 2 kann in ein nicht dargestelltes Gehäuse eines Handgriffs eines Endoskops eingesetzt werden.

In die Seitenwand der Bohrung 3 münden ein Spülmediumeingang 6, ein Spülmediumausgang 7, ein Gaseingang 8 und ein Gasausgang 9. Diese weisen jeweils einen Anschlussstutzen auf, auf den eine entsprechende Anschlussleitung aufgeschoben werden kann. In Fig. 1 sind ein Spülschlauch 10 und ein Insufflationsschlauch 11 gezeigt, die jeweils den proximalen Abschnitt einer Spülleitung bzw. einer Insufflationsleitung bilden und die auf den jeweiligen Anschlussstutzen des Spülmediumeingangs 6 bzw. des Gaseingangs 8 aufgeschoben sind. Der Spülschlauch 10 ist im Betrieb des Insufflations- und Spülventils 1 mit seinem nicht dargestellten Ende an einen Vorratsbehälter für Spülmedium, etwa einen Spülflüssigkeitsbeutel, angeschlossen, während der Insufflationsschlauch 11 mit einem Insufflationsgerät verbunden ist (nicht dargestellt). Das Insufflationsgerät kann beispielsweise eine Insufflationspumpe sein, die ein Insufflationsgas, etwa CO₂, unter einem für die medizinische Insufflation geeigneten Druck zur Verfügung stellt. Der Vorratsbehälter für Spülmedium kann von dem Insufflationsgerät ebenfalls mit Druck beaufschlagt werden, insbesondere mit dem gleichen Druck, unter dem das Insufflationsgas über den Insufflationsschlauch 11 bereitgestellt wird, so dass das Spülmedium mit demselben Druck über den Spülschlauch 10 zugeführt wird.

Der distale Abschnitt der Spül- und der Insufflationsleitung ist in Fig. 1 jeweils nicht dargestellt. An den Anschlussstutzen 7' des Spülmediumausgangs 7 und den Anschlussstutzen 9' des Gasausgangs 9 sind im Betrieb des Insufflations- und Spülventils 1 entsprechende Anschlussleitungen angeschlossen, etwa Schläuche oder auch fest im Endoskop ausgebildete Kanäle, durch die das Spülmedium und das Insufflationsgas durch den Schaft bis zum distalen Endbereich des Endoskops geführt werden. Die schräge Ausrichtung der betreffenden Anschlussstutzen 7', 9' ermöglicht eine platzsparende Anordnung des Spül- und Insufflationsventils 1 und der betreffenden Leitungen bzw. Kanäle. Die Spül- und die Insufflationsleitung können distalseitig des Insufflations- und Spülventils 1 in einen gemeinsamen Kanal münden.

In die Bohrung 3 ist ein äußerer Stößel 20 eingesetzt, der im Wesentlichen rohrförmig mit einer zentralen durchgehenden Längsbohrung 21 ausgebildet ist. Der äußere Stößel 20 ist in der Bohrung durch vier als O-Ring ausgebildete Dichtringe 22, 23, 24, 25 geführt, die jeweils in umlaufenden Nuten auf der Außenseite des äußeren Stößels 20 gehalten sind. Zwischen dem obersten Dichtring 22 und dem zweitobersten Dichtring 23 weist der äußere Stößel 20 auf seiner Außenseite eine umlaufende Nut 26 auf, deren Breite etwa dem axialen Abstand zwischen der Mündung des Spülmediumeingangs 6 und der des Spülmediumausgangs 7 entspricht. Zwischen dem zweitobersten Dichtring 23 und dem zweituntersten Dichtring 24 weist der äußere Stößel 20 einen Durchmesser auf, der nahezu dem Innendurchmesser der Bohrung 3 entspricht; hierdurch bildet der äußere Stößel 20 in diesem Bereich einen Sperrkörper 27 aus, der zum Verschließen der Mündung des Spülmediumausgangs 7 geeignet ist. Zwischen dem zweituntersten Dichtring 24 und dem untersten Dichtring 25 ist in die Außenseite des äußeren Stößels 20 eine weitere Nut 28 eingebracht, die mindestens einen beispielsweise durch eine kurze Querbohrung gebildeten Durchbruch 29 zur Längsbohrung 21 aufweist.

In seinem oberen Bereich bildet der äußere Stößel 20 einen verdickten Stößelkörper 30, auf den eine Kunststoffkappe 31 aufgesetzt ist, die einen Ventilknopf bildet. Die Kunststoffkappe 31 weist an ihrer Oberseite eine flache Einsenkung 32 auf, die eine Betätigungsfläche des Ventilknopfs darstellt. Die zentrale Längsbohrung des äußeren Stößels erstreckt sich durch den Stößelkörper 30 und die Kunststoffkappe 31 bis in die Mitte der Einsenkung 32. Der Stößelkörper 30 ist in der Erweiterung 5 des Ventilkörpers 2 verschiebbar geführt. Auf das obere Ende des Ventilgehäuses 2 ist ein Kopfteil 12 aufgesetzt, das ein mit einem Kunststoffkörper 13 ummanteltes becherförmiges Metallteil 14 umfasst. Der Kunststoffkörper 13 ist mit einer Überwurfmutter 15 am oberen Ende des Ventilgehäuses 2 befestigt. Das Metallteil 14 weist eine zentrale Bohrung auf, durch die der Stößelkörper 30 ragt. Zwischen das Metallteil 14 und die Kunststoffkappe 31 ist eine Schraubenfeder 33 eingesetzt, die den äußeren Stößel 20 in seine obere, vom Boden 4 entfernte Stellung vorspannt.

In der zylindrischen Längsbohrung 21 ist ein innerer Stößel 40 angeordnet. Der innere Stößel 40 umfasst einen zylindrischen Schaft 41, dessen Außendurchmesser nahezu dem Innendurchmesser der Längsbohrung 21 entspricht und der in die Längsbohrung dichtend eingesetzt und in dieser verschiebbar ist. In seinem unteren Bereich weist der Schaft 41 eine umlaufende Nut 42 auf, deren Breite mindestens etwa dem Abstand des Durchbruchs 29 des äußeren Stößels 20 vom unteren Ende des äußeren Stößels 20 entspricht. Das bodennahe, d.h. dem Boden 4 zugewandte Ende des Schafts 41 ist mit einer als Ventilteller 43 ausgebildeten Erweiterung versehen, die oberseitig kegelförmig geformt ist und nach Art eines Tellerventils in eine als Ventilsitz 34 dienende entsprechende kegelförmige Einsenkung im unteren Ende des äußeren Stößels 20 dichtend eingreift.

In der in Fig. 1 gezeigten Stellung ragt das obere, bodenferne Ende des Schafts 41 des inneren Stößels 40 über die Betätigungsfläche, die durch die Einsenkung 32 in der axialen Oberfläche der Kunststoffkappe 31 gebildet wird, hinaus. In dieser Stellung liegt der Ventilteller 43 des inneren Stößels 40 dichtend im Ventilsitz 34 des äußeren Stößels 20 an. Insufflationsgas, das von einem Insufflationsgerät unter einem Druck über den Insufflationsschlauch 11 in den unteren Bereich des Innenraums 16 des Ventilgehäuses 2 zugeführt wird, kann daher nicht in die umlaufende Nut 42 des inneren Stößels 40 und somit nicht in den Gasausgang 9 gelangen. Durch den Druck des Gases im Innenraum 16 wird der Ventilteller 43 in den Ventilsitz 34 gedrückt, so dass der innere Stößel 40 in der in Fig. 1 gezeigten Stellung, in der das obere Ende über die Oberfläche der Kunststoffkappe 31 vorsteht, gehalten wird. Gleichzeitig wirkt der Ventilteller 43 mit dem Ventilsitz 34 als Anschlag zur Begrenzung der Bewegung des inneren Stößels und zur Festlegung der bodenfernen Stellung des inneren Stößels 40 relativ zum äußeren Stößel 20 zusammen.

Der äußere Stößel 20 wird in der in Fig. 1 dargestellten Stellung durch die Schraubenfeder 33 in seiner oberen, d.h. vom Boden 4 entfernten Stellung gehalten, wobei die Aufwärtsbewegung durch den Stößelkörper 30 und das Metallteil 14 begrenzt wird. Zusätzlich wird der äußere Stößel 20 durch den im Innenraum 16 herrschenden Gasdruck in dieser Stellung gehalten. In dieser Position korrespondiert die Nut 26 mit dem Spülmediumeingang 6, jedoch ist zwischen dem Spülmediumeingang 6 und dem Spülmediumausgang 7 der zweitoberste Dichtring 23 angeordnet, so dass keine Verbindung zum Spülmediumausgang 7 besteht. Nach oben ist die Nut 26 durch den obersten Dichtring 22 abgedichtet, so dass ein Verlust von Spülmedium vermieden wird. In der in Fig. 1 gezeigten ersten Stellung des Insufflations- und Spülventils 1, die die Normalstellung, d.h. die unbetätigte Stellung ist, sind somit sowohl die Spülung als auch die Insufflation unterbrochen.

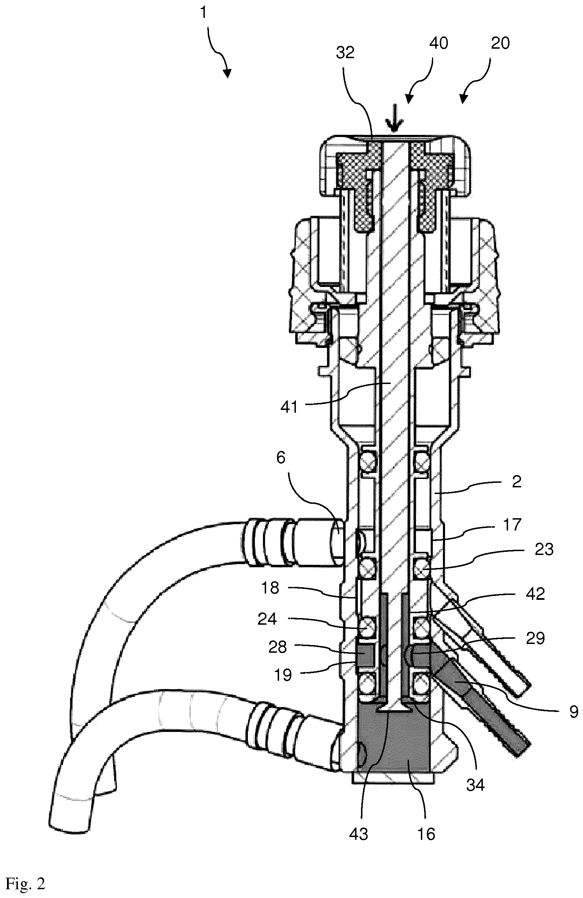

Zum Betätigen des Insufflations- und Spülventils 1 legt der Benutzer auf die durch die flache Einsenkung 32 gebildete Betätigungsfläche des Ventilknopfs einen Finger auf und übt zum Verschieben des inneren Stößels 40 bzw. des äußeren Stößels 20 einen Fingerdruck aus. In der in Fig. 2 gezeigten zweiten Stellung des Insufflations- und Spülventils 1 ist durch Betätigung mit dem Finger der innere Stößel 40 soweit in den äußeren Stößel hineingedrückt (durch den Pfeil angedeutet), dass das obere Ende des Schafts 41 des inneren Stößels 40 mit der Oberfläche des Bodens der Einsenkung 32 bündig abschließt. In dieser Stellung ist der Ventilteller 43 aus dem Ventilsitz 34 herausgedrückt, so dass Insufflationsgas aus dem Innenraum 16 durch die Nut 42 des inneren Stößels 40 und einen Verbindungskanal des äußeren Stößels 20, der durch den Durchbruch 29 und die Nut 28 gebildet wird, in den Gasausgang 9 und weiter zum distalen Ende des Endoskops strömen kann (in Fig. 2 angedeutet). In dieser Stellung kann somit eine Gasinsufflation stattfinden. Während sich der innere Stößel 40 somit in seiner bodennahen Stellung befindet, ist der äußere Stößel wie in Fig. 1 in seiner bodenfernen Stellung, die die Schließstellung ist; eine Spülung findet daher nicht statt.

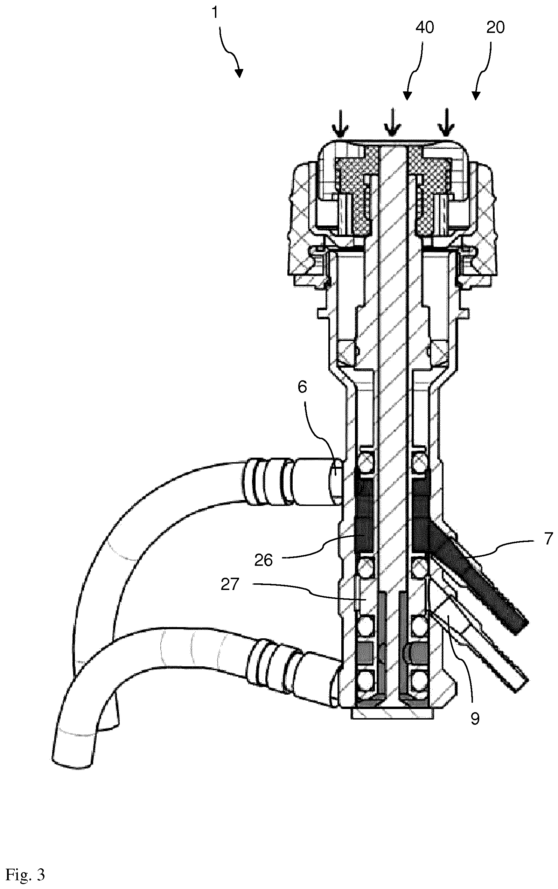

In der in Fig. 3 dargestellten dritten Stellung des Insufflations- und Spülventils 1 ist durch weiteren Druck mit dem Finger von dem Benutzer auch der äußere Stößel 20 nach unten gedrückt worden (durch die drei Pfeile angedeutet). In dieser Position korrespondiert weiterhin die Nut 26 mit dem Spülmediumeingang 6, jedoch ebenso mit dem Spülmediumausgang 7. Es wird somit über die umlaufende Nut 26 des äußeren Stößels 20 eine Verbindung zwischen dem Spülmediumeingang 6 und dem Spülmediumausgang 7 geschaffen, es kann daher Spülmedium aufgrund des Drucks, unter dem dieses im Spülmediumeingang 6 bereitgestellt wird, in den Spülmediumausgang 7 und weiter zum distalen Ende des Endoskops gelangen (in Fig. 3 angedeutet). In dieser Stellung kann somit eine Spülung stattfinden. Der innere Stößel 40 befindet sich dabei wie in Fig. 2 relativ zum äußeren Stößel 20 in seiner bodennahen Stellung, jedoch wird nun der Gasausgang 9 durch den Sperrkörper 27 verschlossen. Eine Insufflation findet daher nicht statt.

Im Bereich des Spülmediumeingangs 6, des Spülmediumausgangs 7 und des Gasausgangs 9 ist jeweils eine ringförmig umlaufende flache Nut 17, 18, 19 in die Wand der ansonsten zylindrischen Bohrung 3 eingebracht (s. Fig. 2). Die Nuten 17, 18, 19 weisen jeweils zumindest teilweise abgeschrägte Ränder auf, so dass die beiden mittleren Dichtringe 23, 24, die über die Ränder gleiten, nicht beschädigt werden. Hierdurch kann eine Beschädigung der Dichtringe 23, 24 beim Gleiten über die Ränder der Mündungen des Spülmediumeingangs 6, des Spülmediumausgangs 7 bzw. des Gasausgangs 9 vermieden werden. Die Wand des Ventilkörpers 2 kann im entsprechenden Bereich verstärkt sein.

In einer in den Figuren nicht gezeigten Zwischenstellung zwischen der zweiten (s. Fig. 2) und der dritten (s. Fig. 3) Stellung des Insufflations- und Spülventils 1 ist eine gleichzeitige Insufflation und Spülung möglich, wobei die jeweiligen Strömungsquerschnitte der Verbindungen zwischen dem Spülmediumeingang 6 und dem Spülmediumausgang 7 bzw. zwischen dem Gaseingang 8 und dem Gasausgang 9 eine feinfühlige Steuerung beider Vorgänge erlauben. Da das Spülmedium und das Insufflationsgas unter einem nahezu gleichen Druck zugeführt werden, tritt dabei nicht in störendem Maße Spülmedium in den Gasausgang 9 ein oder Insufflationsgas in den Spülmediumausgang 7.

Wie aus den Figuren ersichtlich ist, kann durch Lösen der Überwurfmutter 15 das Kopfteil 12 vom Ventilgehäuse 2 gelöst und der äußere Stößel 20 aus der Bohrung 3 entnommen werden. Der innere Stößel 40 kann sodann nach unten aus der Längsbohrung 21 entnommen werden. Das Insufflations- und Spülventil 1 ist daher sehr leicht zerlegbar und kann in zerlegtem Zustand einfach gereinigt werden, da alle zu reinigenden Oberflächen leicht zugänglich sind. Das Insufflations- und Spülventil 1 kann durch eine umgekehrte Abfolge von Schritten ebenso leicht zusammengesetzt werden.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Insufflations- und Spülventil
- 2: Ventilgehäuse
- 3: Bohrung
- 4: Boden
- 5: Erweiterung
- 6: Spülmediumeingang
- 7: Spülmediumausgang
- 7': Anschlussstutzen
- 8: Gaseingang
- 9: Gasausgang
- 9': Anschlussstutzen
- 10: Spülschlauch
- 11: Insufflationsschlauch
- 12: Kopfteil
- 13: Kunststoffkörper
- 14: Metallteil
- 15: Überwurfmutter
- 16: Innenraum
- 17: Nut
- 18: Nut
- 19: Nut
- 20: Äußerer Stößel
- 21: Längsbohrung
- 22: Dichtring
- 23: Dichtring
- 24: Dichtring
- 25: Dichtring
- 26: Nut
- 27: Sperrkörper
- 28: Nut
- 29: Durchbruch
- 30: Stößelkörper
- 31: Kunststoffkappe
- 32: Einsenkung
- 33: Schraubenfeder
- 34: Ventilsitz
- 40: Innerer Stößel
- 41: Schaft
- 42: Nut
- 43: Ventilteller

## Patentansprüche

1. Insufflations- und Spülventil für ein Endoskop, umfassend ein Ventilgehäuse (2) mit einer im Wesentlichen zylindrischen, an ihrem einen Ende mit einem Boden (4) abgeschlossenen Bohrung (3), in die ein Gaseingang (8), ein Gasausgang (9), ein Spülmediumeingang (6) und ein Spülmediumausgang (7) münden, einen äußeren Stößel (20), der in der Bohrung (3) zwischen einer Offenstellung, in der der Spülmediumausgang (7) mit dem Spülmediumeingang (6) fluidverbunden ist, und einer Schließstellung, in der der Spülmediumausgang (7) von dem Spülmediumeingang (6) getrennt ist, verschiebbar ist, und einen inneren Stößel (40), der in einer zum Boden (4) offenen Längsbohrung (21) des äußeren Stößels (20) zum Ermöglichen einer Fluidverbindung des Gasausgangs (9) mit dem Gaseingang (8) in einer bodennahen Stellung und zum Unterbrechen der Fluidverbindung des Gasausgangs (9) mit dem Gaseingang (8) in einer bodenfernen Stellung verschiebbar ist, wobei der Gaseingang (8) bodennäher als der Gasausgang (9) in die Bohrung (3) des Ventilgehäuses (2) mündet, **dadurch gekennzeichnet, dass** die bodenseitige Öffnung der Längsbohrung (21) in der bodenfernen Stellung des inneren Stößels (40) verschlossen ist, wobei das bodenseitige Ende des inneren Stößels (40) mit einem Ventilteller (43) zum Verschließen der bodenseitigen Öffnung der Längsbohrung (21) ausgebildet ist.

2. Insufflations- und Spülventil nach Anspruch 1, **dadurch gekennzeichnet, dass** der Gasausgang (9) und der Gaseingang (8) über einen Verbindungskanal des äußeren Stößels (20), eine auf einer Außenseite des inneren Stößels (40) ausgebildete Ausnehmung und die bodenseitige Öffnung der Längsbohrung (21) verbindbar sind.

3. Insufflations- und Spülventil nach Anspruch 2, **dadurch gekennzeichnet, dass** die auf der Außenseite des inneren Stößels (40) ausgebildete Ausnehmung als umlaufende Nut (42) oder Verjüngung ausgebildet ist.

4. Insufflations- und Spülventil nach einem der vorhergehenden Ansprüche, **dadurch gekennzeichnet, dass** der innere Stößel (40) eine solche Länge aufweist, dass in seiner bodenfernen Stellung ein bodenfernes Ende des inneren Stößels (40) über eine Betätigungsfläche eines Ventilknopfs des äußeren Stößels (20) hinausragt und in seiner bodennahen Stellung mit der Betätigungsfläche des Ventilknopfs bündig abschließt.

5. Insufflations- und Spülventil nach einem der vorhergehenden Ansprüche, **dadurch gekennzeichnet, dass** der Gasausgang (9) in der Offenstellung des äußeren Stößels (20) verschlossen ist.

6. Insufflations- und Spülventil nach einem der vorhergehenden Ansprüche, **dadurch gekennzeichnet, dass** im Bereich der Mündung des Spülmediumeingangs (6), des Spülmediumausgangs (7) und/oder des Gasausgangs (9) die Bohrung des Ventilgehäuses (2) durch eine umlaufende Nut (17, 18, 19) mit mindestens einer abgeschrägten Wand erweitert ist.

7. Insufflations- und Spülventil nach einem der vorhergehenden Ansprüche, **dadurch gekennzeichnet, dass** der Gasausgang (9) durch einen schräg zur Längsachse der Bohrung (3) gerichteten Kanal gebildet wird.

8. Endoskop mit einem Insufflations- und Spülventil (1) nach einem der vorhergehenden Ansprüche.

## Claims

1. Insufflation and irrigation valve for an endoscope, comprising a valve housing (2) with a substantially cylindrical bore (3) which is closed off at one end by a bottom (4) and into which a gas inlet (8), a gas outlet (9), an irrigation medium inlet (6) and an irrigation medium outlet (7) open, an outer plunger (20) which is movable in the bore (3) between an open position, in which the irrigation medium outlet (7) is fluidically connected to the irrigation medium inlet (6), and a closed position, in which the irrigation medium outlet (7) is separated from the irrigation medium inlet (6), and an inner plunger (40) which is movable in a longitudinal bore (21) of the outer plunger (20), open toward the bottom (4), in order to permit a fluidic connection of the gas outlet (9) to the gas inlet (8) in a position near the bottom and in order to interrupt the fluidic connection of the gas outlet (9) to the gas inlet (8) in a position remote from the bottom, wherein the gas inlet (8) opens into the bore (3) of the valve housing (2) at a location nearer the bottom than the gas outlet (9), **characterized in that** the bottom-side opening of the longitudinal bore (21) is closed when the inner plunger (40) is in the position remote from the bottom, wherein the bottom-side end of the inner plunger (40) is designed with a valve disk (43) for closing the bottom-side opening of the longitudinal bore (21).

2. Insufflation and irrigation valve according to Claim 1, **characterized in that** the gas outlet (9) and the gas inlet (8) are connectable via a connection channel of the outer plunger (20), a recess formed on an outer face of the inner plunger (40) and the bottom-side opening of the longitudinal bore (21).

3. Insufflation and irrigation valve according to Claim 2, **characterized in that** the recess formed on the outer face of the inner plunger (40) is designed as a peripheral groove (42) or tapering.

4. Insufflation and irrigation valve according to one of the preceding claims, **characterized in that** the inner plunger (40) has such a length that, in its position remote from the bottom, an end of the inner plunger (40) remote from the bottom protrudes above an actuation surface of a valve button of the outer plunger (20) and, in its position near the bottom, terminates flush with the actuation surface of the valve button.

5. Insufflation and irrigation valve according to one of the preceding claims, **characterized in that** the gas outlet (9) is closed in the open position of the outer plunger (20).

6. Insufflation and irrigation valve according to one of the preceding claims, **characterized in that**, in the area of the mouth of the irrigation medium inlet (6), the irrigation medium outlet (7) and/or the gas outlet (9), the bore of the valve housing (2) is widened by a peripheral groove (17, 18, 19) with at least one beveled wall.

7. Insufflation and irrigation valve according to one of the preceding claims, **characterized in that** the gas outlet (9) is formed by a channel directed obliquely with respect to the longitudinal axis of the bore (3).

8. Endoscope with an insufflation and irrigation valve (1) according to one of the preceding claims.

## Revendications

1. Valve d'insufflation et de purge pour un endoscope, comprenant un corps de valve (2) avec un alésage essentiellement cylindrique (3), fermé à sa première extrémité par un fond (4), et dans lequel débouchent une entrée de gaz (8), une sortie de gaz (9), une entrée de milieu de purge (6) et une sortie de milieu de purge (7), avec un poussoir extérieur (20) qui est déplaçable dans l'alésage (3) entre une position ouverte, dans laquelle la sortie de milieu de purge (7) est en communication fluidique avec l'entrée de milieu de purge (6), et une position fermée, dans laquelle la sortie de milieu de purge (7) est séparée de l'entrée de milieu de purge (6), et avec un poussoir intérieur (40), qui est déplaçable dans un alésage longitudinal (21) ouvert vers le fond (4) du poussoir extérieur (20) de façon à permettre une communication fluidique de la sortie de gaz (9) avec l'entrée de gaz (8) dans une position proche du fond et à interrompre la communication fluidique de la sortie de gaz (9) avec l'entrée de gaz (8) dans une position éloignée du fond, dans lequel l'entrée de gaz (8) débouche plus près du fond que la sortie de gaz (9) dans l'alésage (3) du corps de valve (2),
**caractérisée en ce que** l'ouverture proche du fond de l'alésage longitudinal (21) est fermée dans la position éloignée du fond du poussoir intérieur (40), dans lequel l'extrémité proche du fond du poussoir intérieur (40) est réalisée avec une tête de valve (43) destinée à la fermeture de l'ouverture proche du fond de l'alésage longitudinal (21).

2. Valve d'insufflation et de purge selon la revendication 1, **caractérisée en ce que** la sortie de gaz (9) et l'entrée de gaz (8) peuvent être reliées par un canal de liaison du poussoir extérieur (20), un évidement formé sur le côté extérieur du poussoir intérieur (40) et l'ouverture proche du fond de l'alésage longitudinal (21).

3. Valve d'insufflation et de purge selon la revendication 2, **caractérisée en ce que** l'évidement formé sur le côté extérieur du poussoir intérieur (40) est réalisé sous forme de rainure (42) ou de rétrécissement périphérique.

4. Valve d'insufflation et de purge selon l'une quelconque des revendications précédentes, **caractérisée en ce que** le poussoir intérieur (40) présente une longueur telle que dans sa position éloignée du fond une extrémité éloignée du fond du poussoir intérieur (40) sorte au-delà d'une face d'actionnement d'un bouton de valve du poussoir extérieur (20) et que dans sa position proche du fond il se termine à fleur de la face d'actionnement du bouton de valve.

5. Valve d'insufflation et de purge selon l'une quelconque des revendications précédentes, **caractérisée en ce que** la sortie de gaz (9) est fermée dans la position ouverte du poussoir extérieur (20).

6. Valve d'insufflation et de purge selon l'une quelconque des revendications précédentes, **caractérisée en ce que** dans la région de l'embouchure de l'entrée de milieu de purge (6), de la sortie de milieu de purge (7) et/ou de la sortie de gaz (9) l'alésage du corps de valve (2) est élargi par une rainure périphérique (17, 18, 19) avec au moins une paroi oblique.

7. Valve d'insufflation et de purge selon l'une quelconque des revendications précédentes, **caractérisée en ce que** la sortie de gaz (9) est formée par un canal orienté en oblique par rapport à l'axe longitudinal de l'alésage (3).

8. Endoscope muni d'une valve d'insufflation et de purge (1) selon l'une quelconque des revendications précédentes.
